# EUROPEAN PATENT SPECIFICATION

(11) **EP 1 898 931 B1**
(45) Date of publication and mention of the grant of the patent: **31.12.2014**
(21) Application number: 06757762.7
(22) Date of filing: 07.07.2006
(51) Int. Cl.: A61K 38/28

(54) **GLUCOSE UPTAKE MODULATOR AND METHOD FOR TREATING DIABETES OR DIABETIC COMPLICATIONS**
GLUCOSE-AUFNAHMEMODULATOR UND VERFAHREN ZUR BEHANDLUNG VON DIABETES ODER DIABETISCHEN KOMPLIKATIONEN
MODULATEUR DE CAPTAGE DU GLUCOSE ET PROCEDE DE TRAITEMENT DU DIABETE ET DES COMPLICATIONS DU DIABETE

(30) Priority: 07.07.2005 US 595457 P
(43) Date of publication of application: 19.03.2008
(73) Proprietor: Postech Foundation, Pohang-City, Kyungsangbuk-do 790-784 (KR); Posco, Pohang-shi, Kyungsangbuk-do 790-300 (KR)
(72) Inventor: YEA, Kyung-Moo, San 31 Hyojadong, Pohang Gyeongsangbuk-do 790-784 (KR); KIM, Jae-Yoon, Pohang-city, Kyungsangbuk-do 790-784 (KR); Kim, Jong-Hyun, Bethesda, MD 20852 (US); LEE, Byoung-Dae, San 31 Hyojadong, Pohang Gyeongsangbuk-do 790-784 (KR); LEE, Seung-Je, San 31 Hyojadong, Pohang Gyeongsangbuk-do 790-784 (KR); LEE, Tae-Hoon, San 31 Hyojadong, Pohang Gyeongsangbuk-do 790-784 (KR); SUH, Pann-Ghill, Pohang-city, Kyungsangbuk-do 790-784 (KR); RYU, Sung-Ho, Pohang-city, Kyungsangbuk-do 790-784 (KR)
(74) Representative: Winter, Brandl, Fürniss, Hübner, Röss, Kaiser, Polte - Partnerschaft mbB
(86) International application number: PCT/KR2006/002656
(87) International publication number: WO 2007/007982

(56) References cited:
- WO-A1-2004/080481
- US-A- 3 197 368
- US-B1- 6 846 801
- SOGA T. ET AL.: 'Lysophosphatidylcholine enhances glucose-dependent insulin secretion via an orphan G-protein-coupled receptor' BIOCHEMICAL AND BIOPHYSICAL RESEARCH COMMUNICATIONS vol. 326, no. 4, 28 January 2005, pages 744 - 751, XP004682876

## Description

### CROSS-REFERENCE TO RELATED APPLICATION

This application claims priority to and the benefit of US provisional patent application No. 60/595,457 filed in the United State of America Patent & Trademark Office on July 7, 2005.

### BACKGROUND OF THE INVENTION

### (a) Field of the Invention

The present invention relates to a glucose uptake modulator for treating a diabetes or diabetic complications in a mammal in need thereof, which comprises administering to said mammal an effecting amount of the glucose uptake modulator.

### (b) Description of the Related Art

In addition to insulin, various hormones or physiological conditions are capable of stimulating the glucose uptake. For example, exercise induces glucose uptake in skeletal muscle through an insulin independent pathway. Also, activation of α₁-adrenergic or endothelin_{A} receptors result in enhanced glucose uptake rates independent of insulin. Some of the signaling mechanisms that mediate these metabolic responses are similar to those utilized by insulin, whereas others are clearly distinct. For instance, the stimulation of glucose uptake that occurs in adipocytes treated with arachidonic acid, peroxisome proliferators activated receptor γ agonists seems to involve specific and insulin-independent signaling pathways.

For many years adipose tissue was viewed as playing a key role in total body lipid and energy homeostasis. Removal of excess glucose from the circulation involves the stimulation of glucose transport into adipose and muscle tissue. It has become clear that glucose intolerance in type 2 diabetes is manifested by defects in glucose transport into adipose tissue. Therefore, the finding of new endogenous factors which regulate glucose transport in adipocytes is essential for our understanding of diabetes process and for the development of improved therapeutic strategies.

Bioactive molecules such as hormones, neurotransmitters, and cytokines play important roles in many regulatory processes in an organism. These molecules have essential functions in intercellular communication. Moreover, they have been used to diagnose and treat human diseases. To find novel bioactive molecules, traditionally, sequential column-chromatography has been used. However, there was inevitable limitation in the low abundance of the molecules of interest by low yield due to the many column steps.

To solve this problem, previously, the present inventors developed a new integrative method, Ligand Profiling and Identification (LPI), for searching various endogenous ligands. This method, based on parallel column chromatography methods and sensitive MS analysis, is suitable for searching low abundance bioactive molecules rapidly and simultaneously. Recently, for the efficient purification, we evolved this LPI technology by adding the protease filtering method. We assumed that these systematic and sensitive analytical techniques could be effectively used for the identification of novel bioactive molecules from tissues or body fluids.

These prior art references do not specifically describe or suggest combining an insulin sensitizer with an anorectic, and effects of such combination. Development of excellent drugs which are sufficiently improved as a medicine having an excellent diabetic treatment effect without apparent detection of side effects is desired.

### SUMMARY OF THE INVENTION

An object of the present invention is to provide a glucose uptake stimulator which comprises lysophosphatidic acid (LPA). Lysophosphatidylcholine, lysophosphatidylserine, and lysophosphatidic acid activates a glucose uptake without insulin. Urocortin acts as co-factor for insulin action in the regulation of glucose homeostasis.

Another object of the present invention is to provide a pharmaceutical composition which comprises lysophosphatidic acid. The pharmaceutical composition further comprises a pharmaceutically acceptable carrier, diluent or exipient. In addition, the pharmaceutical composition further comprises at least a compound selected from the group consisting of insulin secretion enhancers, biguanides, and α-glucosidase inhibitors.

A still further object of the present invention is to provide a method for treating diabetes or diabetic complications in a mammal in need thereof, which comprises administering to said mammal an effecting amount of the insulin sensitizer lysophosphatidic acid. The diabetic complication is obesity, hyperlipidemia, arteriosclerosis, hypertension or heart disease. In addition, the method comprises a step of administering to said mammal an effecting amount of an insulin sensitizer in combination of at least a compound selected from the group consisting of insulin secretion enhancers, biguanides, and α-glucosidase inhibitors.

### BRIEF DESCRIPTION OF THE DRAWINGS

FIG. 1A to 1E show an identification of a novel glucose uptake stimulating molecule from serum.
FIG. 8A and 8B shows LPA stimulating glucose uptake in 3T3-L1 adipocytes with dose- and time-dependent manner.
FIG. 9A and 9B shows LPA stimulating glucose uptake in 3T3-L1 adipocytes via LPA receptor and Gαi activation.
FIG. 10A and 10B shows LPA stimulating glucose uptake in 3T3-L1 adipocytes by PI3-kinase dependent signaling pathway.
FIG. 11A to 11D shows LPA lowering the level of blood glucose in normal mouse via LPA receptor activation

### DETAILED DESCRIPTION OF THE EMBODIMENTS

An exemplary embodiment of the present invention will hereinafter be described in detail with reference to the accompanying drawings.

By a glucose uptake modulator is meant any agent which will lower blood glucose levels by increasing the responsiveness of the tissues to insulin.

By patients susceptible to insulin resistant hypertension is meant a patient who exhibits insulin resistance and is therefore likely to exhibit hypertension. Such patients are well known and readily determinable by a physician of ordinary skill in the art.
By treatment is meant any lowering of blood pressure caused by insulin resistance and/or high circulating insulin levels. By prevention is meant partial to total avoidance of hypertension in insulin resistant patients, depending on the severity of the disease.

By "unit dose" is meant a discrete quantity of a glucose uptake modulator in a form suitable for administering for medical or veterinary purposes. Thus, an ideal unit dose would be one wherein one unit, or an integral amount thereof, contains the precise amount of glucose uptake modulator for a particular purpose, e.g., for treating or preventing obesity resulting from treatment with anti-diabetic drugs. As would be apparent to a person of ordinary skill in pharmaceutical formulations, glucose uptake modulator can be formulated into conventional unit doses. These unit doses can be packaged in a variety of forms, e.g., tablets, hard gelatin capsules, foil packets, glass ampules, and the like. Similarly, a unit dose may be delivered from a medicine dropper or from a pump spray. These various unit doses may then be administered in various pharmaceutically acceptable forms of liquid administration, i.e., orally or parenterally. Thus, for example, the contents of a foil packet may be dissolved in water and ingested orally, or the contents of a glass vial may be injected. Similarly, a discrete amount form such as a medicine dropper or a pump spray may be dissolved in water.

By "mammal" is meant any of a class (Mammalia) of higher vertebrates comprising man and all other animals that nourish their young with milk secreted by mammary glands and have the skin usually more or less covered with hair. Especially included in this definition are human beings, whose endurance, stamina or exercise capacity is less than optimal. Such human and non-human animals are readily diagnosed by a physician or veterinarian of ordinary skill.

Glucose homeostasis is maintained by the fine orchestration of hepatic glucose production and cellular glucose uptake. If our body fails to maintain glucose homeostasis, we can be under hyperglycemia or various metabolically disturbed conditions. In the search for novel factor which enhances glucose uptake in 3T3-L1 adipocytes, we applied new integrative method which is based on systematic parallel column chromatography, protease filtering and sensitive MS analysis and identified LPC (not belonging to the present invention).

Lysophospholipids regulate a variety of biological processes including cell proliferation, tumor cell invasiveness, and inflammation. LPC, produced by the action of Phospholipase A₂ (PLA₂) is a major plasma lipid component and transports fatty acids and choline to tissues. It is also known that LPC is highly related in the regulation of glucose homeostasis. Recently, it is has been shown that LPC enhances glucose-dependent insulin secretion from pancreatic-cells. One of LPC's reported physiological action is the induction of insulin secretion from pancreatic cells. Recently, Takatoshi *et al.* identified an orphan G-protein coupled receptor, GPR 119 as a novel Gs-protein coupled receptor for LPC. The GPR 119 is predominantly expressed in pancreatic cells and that activation of GPR 119 by LPC leads to glucose-dependent insulin secretion.

LPA has emerged as a potent and pleiotropic bioactive phospholipid known to regulate a number of cellular events via specific G protein-coupled receptors. LPA can regulate platelet aggregation, actin cytoskeleton activation, fibroblast proliferation, and neurite retraction. Two major pathways have been postulated for the extracellular production of LPA. As a first pathway LPA is released by activated platelets Second pathway: LPA is produced from lysophospholipids by autotaxin (lyso-PLD). Recently, it was reported that LPA is produced in the extracellular medium of adipocytes as the result of the secretion of autotaxin. LPA could be involved in the developmental control of adipose tissue which has key roles in regulating overall energy balance.

As a one of bioactive lysophospholipid, lysophosphatidylserine (LPS) (not belonging to the present invention) is thought to be related in immunological regulation. However, the effects of LPS on cellular activities and the identities of its target molecules have not been fully elucidated. LPS has also been found in ascites of ovarian cancer patients. It has been reported to induce transient increases in intracellular calcium concentration in ovarian and breast cancer cell lines. LPS also stimulated interleukin-2 production in Jurkat T cells, showing inhibitory effect on Jurkat cell proliferation. Furthermore, LPS treatment enhanced nerve growth factor-induced histamine release in rat mast cells and nerve growth factor-induced differentiation of PC 12 cells. Since limited reports have demonstrated the role of LPS in the modulation of some biological responses, its role in various cellular activities and its action mechanism should be investigated.

Besides LPC, many lysophospholipids (LPL) are known to have diverse physiological and pathological functions. However, there is no report that they are involved in regulation of glucose homeostasis. As an endogenous lipid which related in glucose metabolism, dehydroepiandrosterone (DHEA) has been reported. Although, recent studies have demonstrated that DHEA increases glucose uptake rates in adipocytes, there is no report that its effectiveness on animal model. Therefore, we suggest that LPC might be the first endogenous lipid which regulates the level of blood glucose in the diabetic models of mice as well as in normal mice.

For the finding of a novel active ligand, we previously devised new methodology named LPI which is based on the concept of parallel HPLC and active fraction profiling by MS analysis. The parallel HPLC is effective on identification of active molecules by increasing yields as described in previous report. In this work, we added protease filtering method to the parallel HPLC for the more effective purification. Protease is commonly used for protein mapping or protein identification, but the protease filtering method utilizes protease as a purification tool like a column chromatography. Especially, protease filtering is appropriate for exclusion of the inactive peptides which have similar physicochemical properties with active molecule. Although, the inactive peptides are not easily removed by common sequential chromatographies, cleavage of inactive peptides by protease treatment gives rise to the structural changes in inactive peptides and segregation from active molecule by next column chromatography. By combining this protease filtering method and parallel HPLC, we have devised a new ligand identification method and identified LPC with less effort. Therefore, this integrative method may be useful for searching various bioactive molecules, like an orphan GPCR study, with small amount of starting materials.

The stimulation of glucose uptake in 3T3-L1 adipocytes and blood glucose lowering in mice by LPC treatment are sensitive to variations in the acyl chain lengths of LPC. While palmytoyl LPC and myristoyl LPC enhanced glucose uptake in 3T3-L1 adipocytes, stearoyl LPC was ineffective on stimulating glucose uptake in 3T3-L1 adipocytes. When several lysophospholipids, which are structurally different only in polar head group from palmytoyl LPC, were treated to 3T3-L1 adipocytes, there was no stimulation of glucose uptake. This structural specificity of LPC is also confirmed in mouse models. These results suggest that both acyl chain length and phosphatidylcholine head group are critical for stimulation of glucose uptake in 3T3-L1 adipocytes and lowering the level of blood glucose in mice.

Based on the rapid onset and structural specificity in LPC action, the present inventor speculates that the biological activity of LPC may be explained by LPC binding to a specific LPC receptor at the cell surface. Several lysophospholipids have been reported to be ligand for this GPCR family. LPC was reported as a direct ligand that binds and activates G2A and GPR4. However, recently, it was reported that LPC can activate but dose not bind directly G2A and GPR4 in other independent studies. Thus it remains an open question as to whether LPC stimulates glucose uptake via directly binding to G2A and GPR4 or indirectly via another unknown pathway.

The involvement of PKCζ activation in promotion of glucose uptake in adipocytes and muscle cells has long been recognized, but PKCδ activation also controls glucose transport. The involvement of PKCδ in glucose transport activation was originally elucidated in studies using pharmacological agents and insulin. Stimulation of the translocation of GLUT4 to the plasma membrane and glucose uptake by insulin was inhibited by rottlerin in rat skeletal muscle cells. Moreover, overexpression of PKCδ induced the translocation of GLUT4 to the plasma membrane and increased basal glucose uptake to levels attained by insulin. In this study, LPC-induced enhancement of glucose uptake was blocked by rottlerin and the expression of dominant negative PKCδ. However, the pretreatment of conventional PKC inhibitor Gö6976 or the expression of dominant negative PKCζ was shown to have no effect on LPC-stimulated glucose uptake. These findings suggest that only PKCδ is essential for the LPC-stimulated glucose uptake.

One of LPC's reported physiological action is the induction of insulin secretion from pancreatic β-cells. Recently, Takatoshi *et al.* identified an orphan G-protein coupled receptor, GPR 119 as a novel Gs-protein coupled receptor for LPC(Soga, T., et al., Biochem Biophys Res Commun 326, pp744-751, 2005). The GPR 119 is predominantly expressed in pancreatic β-cells and that activation of GPR 119 by LPC leads to glucose-dependent insulin secretion. In this study, we administrated LPC to mice under fasting condition. We also observed that there is no change in concentration of serum insulin after LPC administration to mice. These suggest that the blood glucose lowering in mice is not mediated by insulin secretion but by the direct function of LPC after LPC stimulation.

In summary, our present study shows that LPC stimulate glucose uptake in 3T3-L1 adipocytes. This effect is mediated by PI 3-kinase independent, PKCδ dependent signaling pathway. Moreover, LPC directly lowers the level of blood glucose in diabetic mice models. This new discovery of the blood glucose lowering function of LPC may shed new light on glucose homeostasis and other aspects of glucose metabolism-related biology. The relationship between LPC and metabolic syndrome also merit further investigation. Finally, our results raise the high possibility that LPC may be a useful target for the development of drug therapies for diabetes.

Insulin has been known as major glucose regulator in blood. However, for efficient and fine regulation of blood glucose level, it has been suggested to be need of co-factors for insulin functions. These co-factors may have different functional weights between physiological and pathological conditions. In normal physiological condition, insulin has a role as major glucose regulator and so the co-factors may be aside in the regulation of glucose homeostasis. But in pathological condition, such as diabetes and obesity, the insulin action is highly attenuated and the co-factors may have a great portion of glucose regulation by enhancing insulin action.

A pharmaceutical composition of the present invention can be used as an agent for preventing or treating diabetes or diabetic complications. Examples of the diabetes include insulin-dependent diabetes mellitus, insulin-independent diabetes mellitus and etc. Further, a pharmaceutical composition of the present invention can be used as an agent for preventing or treating diabetic complications (e.g., neuropathy, nephropathy, retinopathy, macroangiopahty, coronary artery diseases, osteopenia, etc.). Further, a pharmaceutical composition of the present invention can be used as an agent for treating impaired glucose tolerance.

Further, use of a pharmaceutical composition of the present invention in combination with insulin secretion enhancers, biguanides, α-glucosidase inhibitors, and etc. provides a more excellent blood sugar lowering effect.

Dosage forms of a pharmaceutical composition of the present invention or its respective active ingredients include oral dosage forms such as tablets, capsules (including soft capsules and microcapsules), powders, granules, syrups, and etc.; and non-oral dosage forms such as injections (e.g., subcutaneous injections, intravenous injections, intramuscular injections, intraperitoneal injections, etc.), external application forms (e.g., nasal spray preparations, transdermal preparations, ointments, etc.), suppositories (e.g., rectal suppositories, vaginal suppositories, etc.), pellets, drip infusions, and etc.

The dosage of a pharmaceutical composition of the present invention may be appropriately determined with reference to the dosage recommended for the respective drug(s), and can be selected appropriately according to the subject, the age and body weight of the subject, current clinical status, administration time, dosage form, method of administration, combination of the drug(s), and etc. The dosage of an insulin sensitizer and an anorectic can be selected appropriately based on clinically used dosage. For administration of an insulin sensitizer to an adult diabetic patient (body weight: 50 kg), for instance, the dose per day is usually 0.01 to 1000 mg, preferably 0.1 to 500 mg. This dose can be administered once to several times a day.

The present invention is further explained in more detail with reference to the following examples. These examples, however, should not be interpreted as limiting the scope of the present invention in any manner.

### EXAMPLE

### METHOD & MATERIAL

**Materials:** Synthetic 14:0, 18:0, 18:1 LPC, insulin and streptozotocin (STZ) were obtained from Sigma (St. Louis, MO). Other lysophospholipids were purchased from Avanti polar lipids. All lipids were dissolved in MeOH as a 50mM stock. All lipid stocks were stored under nitrogen at -70°C in glass vials as single-use aliquots and used within a month. Gö6976 and rottlerin were from Calbiochem. Antibodies were purchased from the following sources: Polyclonal anti-GLUT4 antibody was from Biogenesis Ltd (Sandown, NH). Anti-phospho-Ser473 AKT1 antibody was from Sigma. Anti-phospho-Tyr989 IRS1 was produced in our laboratory. [¹⁴C] 2-deoxy-D-glucose (300mCi/mmol) was purchased from Moravek Biochemicals. Trypsin was from Roche (Mannheim Germany). Tissue culture media and fetal bovine serum were obtained from GIBCO. All other reagents were of analytical grade.

**Cell culture:** 3T3-L1 fibroblasts were grown to confluence in DMEM containing a high glucose concentration, 10% fetal bovine serum, 50U of penicillin per ml, and 50ug of streptomycin per ml and maintained in a 5% CO₂ humidified atmosphere at 37°C. 3T3-L1 was induced to differentiate into adipocytes, as described previously (van den Berghe, N., et al., Mol Cell Biol 14, pp.2372-2377, 1994).

**Animals.** Male ICR mice were purchased from hyochang science (ROK). C57BLKSJ-db/db mice were purchased from SLC (Japan). After intravenous injection of LPC, blood glucose was measured regularly with a portable glucose meter (Gluco-Dr, ROK) after tail snipping. For measurement of serum insulin, blood samples of mice were determined with the insulin-RIA Kit (LINCO, Missouri). Insulin deficient mice were induced in male ICR mice by two consecutive daily intraperitoneal injection of STZ (200 mg/kg) dissolved in sodium citrate (pH 5.5). On the third day after the last STZ injection, acute glucose lowering effect was analyzed after intravenous injection of vehicle, LPC or insulin as described above.

**HPLC purification.** Approximately 350 ml of fresh human serum was mixed with 70% (v/v) acetone, 1 M acetic acid, and 20mM HCl and was centrifuged at 20,000g for 30 min at 4°C. The resultant supernatant was collected and extracted three times with diethyl ether. The aqueous phase was centrifuged at 20,000g for 30 min at 4°C, and the supernatant was loaded onto cartridges of SepPak C18 (Waters) for pre-clearing. Eluent was directly loaded onto a C 18 reverse-phase HPLC column (Vydac 218TP1022, 22 mm x 250 mm). 10 ml fractions were collected, and ∼1% of each fraction was assayed for glucose uptake in 3T3-L1 adipocytes. The active fractions were trypsinized for 12 hr at 37°C and applied with equal amount to a C4 reverse-phase HPLC column (Vydac 214TP5215, 2.1 mm x 150 mm) and a cation-exchange HPLC column (Amersham Pharmacia Min-S HR 5/5, 4.6 mm x 50 mm) each.

**Mass spectrometry and data analysis.** ESI-MS and tandem mass spectrometry (MS/MS) analyses were performed using QSTAR PULSAR I hybrid Q-TOF MS/MS (Applied Biosystems/PE SCIEX, Toronto, Ontario) equipped with a nano-ESI source. The samples were dissolved in 0.1% trifluoroacetic acid delivered into the ESI source using a protana nanospray tip (Odense, Denmark). All of the masses detected by QSTAR were calculated using Analyst QS software provided by Applied Biosystems (AB). The QSTAR was operated at a resolution of 8,000-10,000 with a mass accuracy of 10-30ppm using external calibration maintained for 24h. The voltage of the spray tip was set at 2300V. To identify the common mass by mass information, combined online-database; Dictionary of Natural Products (Chapman &Hall/CRC) was used.

**Glucose uptake measurement.** For measuring glucose uptake in 3T3-L1 adipocytes, cells were grown in serum-free DMEM for 16h and then incubated in the absence or presence of insulin or lysophospholipids for the indicated times at 37°C. Uptake was measured by adding 1µCi of [¹⁴C] 2-deoxy-D-glucose and 3 mM 2-deoxy-D-glucose. After 10 min, the assay was terminated by two quick washes with ice-cold PBS. Cells were lysed in 0.5 ml of lysis buffer containing 0.5 N NaOH and 0.1% SDS. The cell lysates were used for liquid scintillation counting and nonspecific uptake was assayed in the presence of 10 µM cytochalasin B(van den Berghe, N., et al., Mol Cell Biol 14, pp.2372-2377, 1994).

**Membrane fractionation of adipocytes.** For obtaining total membranes (TM) from 3T3-L1 adipocytes, cells were collected into 10 ml of ice-cold HES buffer (250 mM sucrose, 1 mM EDTA, 1 mM phenylmethylsulfonyl fluoride [PMSF], 1 µM pepstatin, 1 µM aprotinin, 1 µM leupeptin, and 20 mM HEPES, pH 7.4) and subsequently homogenized with 30 strokes in a glass Dounce homogenizer at 4°C. After centrifugation at 1,000g for 5 min at 4°C to remove unbroken cells, the supernatant was then centrifugated at 212,000g for 90 min at 4°C to yield a pellet of total cellular membranes. To obtain the plasma membrane (PM) subcellular fraction from 3T3-L1 adipocytes, differential ultracentrifugation was used as described previously (Perrini, S., et al., Diabetes 53, pp.41-52, 2004).

**Adenoviral transfection of PKC isoforms.** The adenovirus expression vector for PKCδ or ζ recombinant adenoviruses has been described previously. After differentiation of cultured 3T3-L1 adipocytes, the culture medium was aspirated and culture was infected with the viral medium containing PKCδ or ζ recombinant adenoviruses for 24 h. The cultures were then washed twice with DMEM and refed. Cells 48 h post-infection were used for glucose uptake or Immunoblotting.

**Immunoblotting.** For preparing total cell lysates, 3T3-L1 adipocytes were washed with Ca²⁺/Mg²⁺-free PBS and then lysed in the lysis buffer (50 mM HEPES, pH 7.2, 150 mM NaCl, 50 mM NaF, 1 mM Na₃VO₄, 10% glycerol, 1% Triton X-100). The lysates were centrifuged at 15,000 rpm for 15 min at 4°C. The proteins were denatured by boiling in laemmli sample buffer for 5 min at 95°C, separated by SDS-PAGE. SDS-gel was transferred to nitrocellulose membrane using Hoefer wet transfer system. Membranes were blocked in TTBS (20 mM Tris-HCl, pH 7.6, 150 mM NaCl, 0.05% Tween 20) containing 5% skimmed milk powder for 30 min and then incubated with antibodies for 3 hours. After washing membranes several times with TTBS, the blots were incubated with HRP (Horseradish peroxidase)-conjugated goat anti-rabbit for 1 hour. The blots were washed with TTBS and developed by ECL.

**Statistical analysis.** All data are expressed as mean ± SE. Statistical analysis was performed by Student's *t* test. **P*<0.01 was considered to indicate statistical significance.

### EXAMPLE 1

### Identification of lysophosphatidylcholine (not belonging to the present invention) as a glucose uptake stimulating molecule from human serum in 3T3-L1 adipocytes.

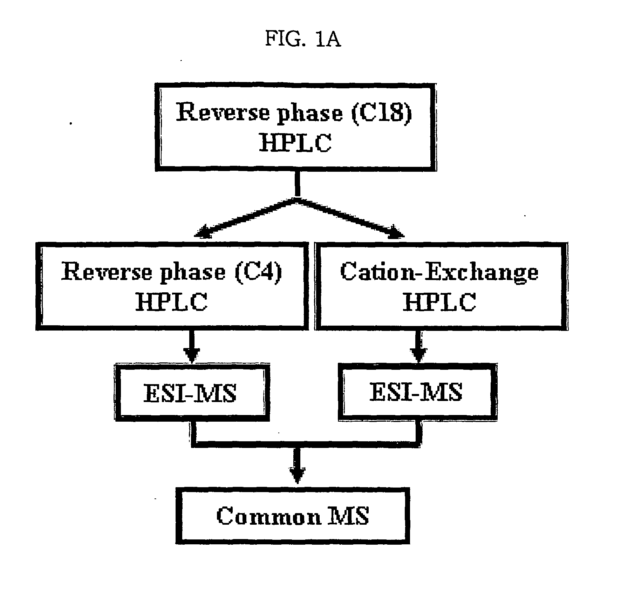

To investigate endogenous factors which stimulate glucose uptake in 3T3-L1 adipocytes, we used a new integrative method which is based on systematic parallel column chromatography, protease filtering method and sensitive MS analysis (Fig. 1A). The fundamental principle of parallel HPLC is that it uses profiling analysis to identify target molecules instead of traditional, sequential purification (Baek, M. C., et al., Proteomics 6, pp,1741-1749, 2006). Low yield by multi-step, sequential columns is a critical limitation of purification because yields after each column are reduced exponentially as purification progresses. This new method minimizes the sequential HPLC steps and utilizes partially purified HPLC fraction for the identification of target molecules, only small amounts of starting material is required compared to the multi-step, sequential HPLC.

In addition to parallel HPLC, we used protease filtering method for efficient purification. If an active fraction doesn't lose its activity after treatment of specific protease, then we can get the fraction which contains active molecule and is drastically separated from various inactive peptides by following column chromatography. Therefore, this method is useful for the purification of non-peptide molecules such as lipids, amines and carbohydrates. With this new integrative method, first, we fractionated acetone extract from human serum (350 ml) by C18 reverse phase (C18) HPLC. Then these HPLC fractions were treated to 3T3-L1 adipocytes and glucose uptake was measured by determining the increase of [14C] 2-deoxy-D-glucose uptake (van den Berghe, N., et al., Mol Cell Biol 14, pp.2372-2377, 1994).

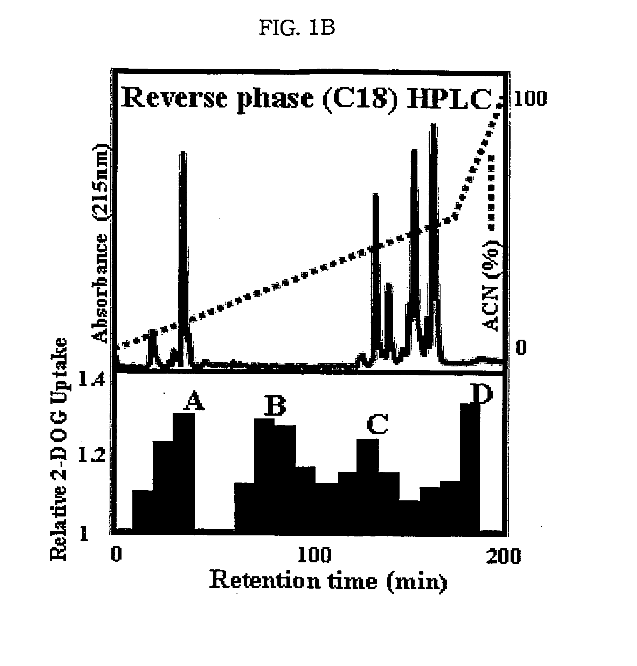

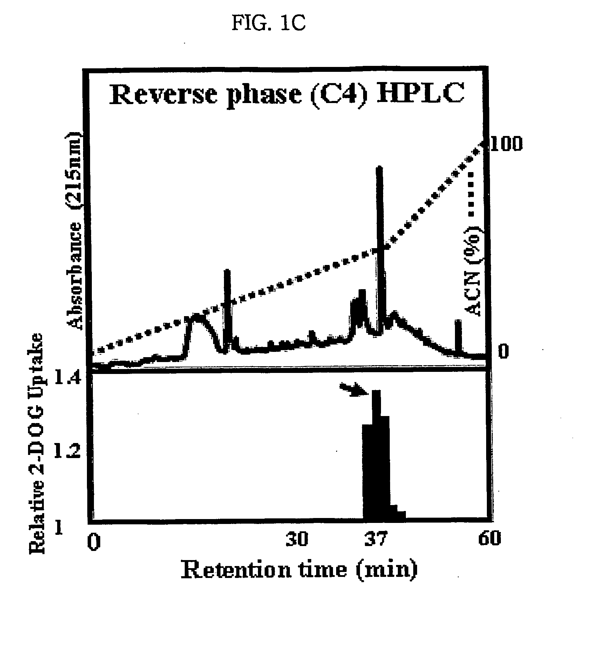

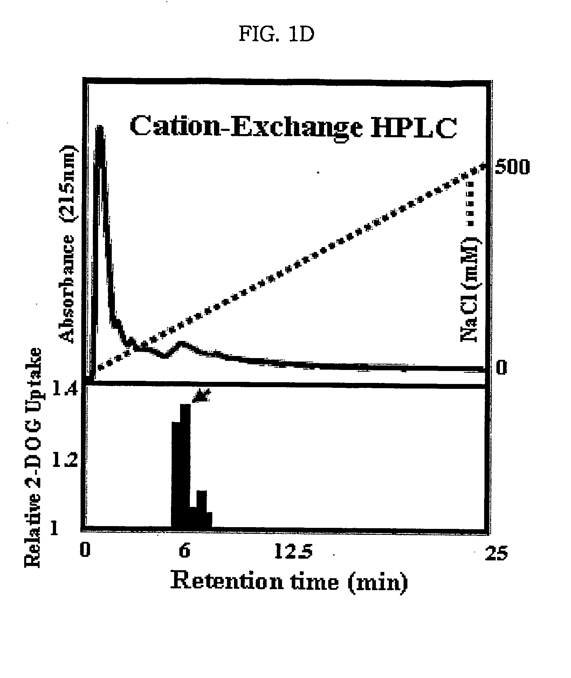

As shown in Fig. 1B, there were at least four kinds of active fractions (A-D) and we tested if their activities are reduced by trypsin treatment. Only the activity of fraction D was not influenced by trypsin treatment, so the fraction D is trypsinized and further separated by C4 reverse phase (C4) and Cation-exchange (SCX) HPLC in parallel. All the fractions of C4 and SCX are screened by measuring the glucose uptake from 3T3-L1 adipocytes (Fig. 1C and 1D).

The active fractions from each column (37 min from C4, 6 min from SCX) are analyzed by ESI-QTOF mass spectrometer. To find common mass, each mass spectrum was compared and there was only one common mass value of 495.33 as a monoisotopic mass (Fig. 1E-upper panel and Fig. 1E-middle panel). With this mass information, we searched combined online-database (*Dictionary of Natural Products)* and identified as palmytoyl lysophosphatidylcholine (LPC). To confirm whether the target molecule is LPC, we analyzed the each fragmentation pattern of standard LPC and 495.33 mass in MS/MS spectrum (Fig. 1F). The standard LPC product-ion spectrum in the positive-ion mode displays several ions originated from the collision-induced dissociation of the phosphocholine head group, including the most intense peak at m/z 183 (Fig. 1F-bottom panel and 1G). The fragmentation pattern of 495.33 mass from C4 and SCX was exactly matched with standard LPC (Fig. 1F-upper panel and 1F-middle panel). Based on the physical properties stated above, we concluded that the active substance is a LPC.

FIG. 1A to 1E show an identification of a novel glucose uptake stimulating molecule from serum. FIG. 1A shows schematic representation of identification strategy for the serum factor which can stimulate glucose uptake in 3T3-L1 adipocytes. FIG. 1B shows C18 reverse-phase HPLC (Vydac 218TP1022, 22 mm x 250 mm) elution profile of the serum. Relative 2-deoxy-D-glucose uptake is expressed as a ratio of the increment obtained by each fraction treatment versus vehicle treatment in 3T3-L1 adipocytes. Active fraction D was arbitrarily selected and trypsinized for further purification. FIG. 1C shows C4 reverse-phase HPLC (Vydac 214TP5215, 2.1 mm x 150 mm) elution profile of the fraction D. FIG. 1D shows a cation-exchange HPLC (Amersham Pharmacia Mini-S HR 5/5, 4.6 mm x 50 mm) elution profile of the fraction D. FIG. 1E shows Mass analysis by ESI-TOF mass spectrometer. Mass spectrum of active fraction of FIG. 1C(top), FIG. 1D(middle) and standard palmytoyl (16:0) LPC (*bottom*). FIG. IF shows a pattern analysis in mass fragmentation and MS/MS spectrum of 495.33 mass in each mass spectrum of FIG. 1E.

### EXAMPLE 2

### Effects of LPC (not belonging to the present invention) on the glucose uptake in 3T3-L1 adipocytes.

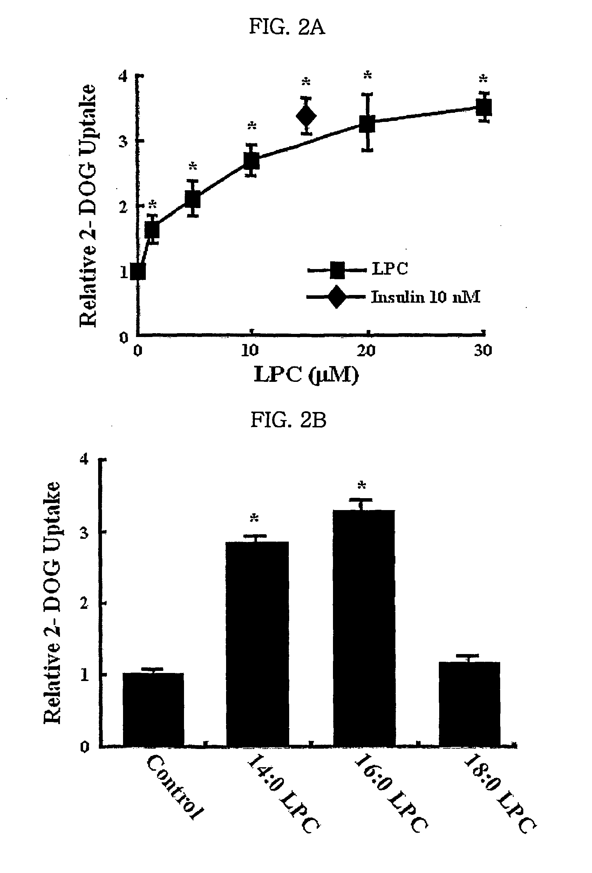

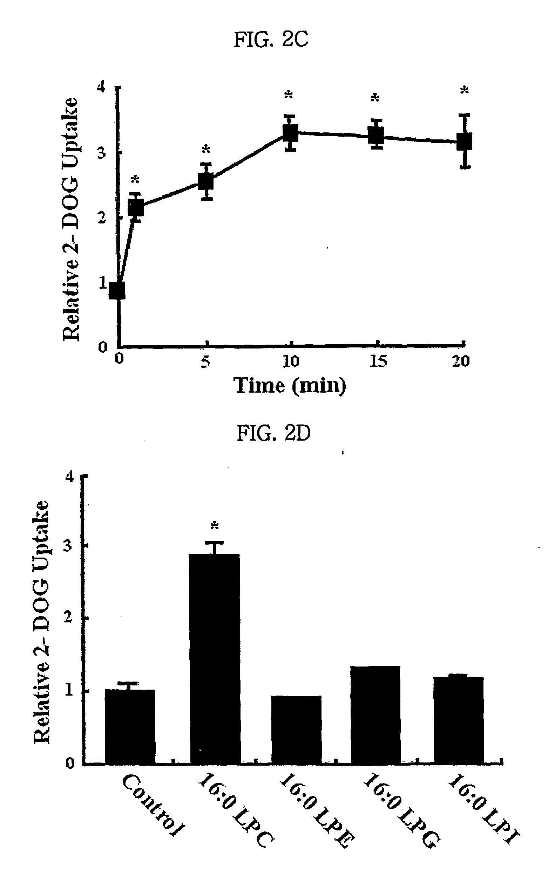

For investigating the effects of LPC on the glucose uptake, 3T3-L1 adipocytes were incubated in the presence of various concentrations of standard LPC for different times. LPC stimulated a time- and dose-dependent increase in glucose uptake in 3T3-L1 adipocytes. An initial statistically significant effect of LPC on glucose uptake was observed at the concentration of 1 µM and the maximal effect at 20 µM (Fig. 2*A*). With 20 µM LPC, glucose uptake was maximally increased after 10 min of incubation with LPC (Fig. 2B). This concentration of LPC was not cytotoxic and was below the critical micellar concentration of 40 to 50 µM (Chaudhuri, P., et al., Circ Res 97, 674-681, 2005).

It is known that the skeletal muscle plays a central role in glucose metabolism, and impairment in glucose metabolism in the skeletal muscle often results in diabetes (Petersen, K. F., et al., Am J Cardiol 90, 11G-18G, 2002; Beck-Nielsen, H., et al., Diabetologia 37, pp217-221, 1994). Although this report mainly focuses on the 3T3-L1 adipocytes, we also found that LPC increased the rate of glucose uptake in a dose dependent manner in C2C12 muscle cells (data not shown). These results imply that LPC may play a role in glucose regulation in both adipocytes and muscle cells.

To determine whether variations in the acyl chain lengths of LPC could affect glucose uptake, several LPC species were tested. Interestingly, myristoyl LPC, palmytoyl LPC stimulated glucose uptake, whereas, stearoyl LPC did not stimulate glucose uptake in 3T3-L1 adipocytes (Fig. 2C). For assessing whether other lysophospholipids could enhance glucose uptake in 3T3-L1 adipocytes, several lysophospholipids were treated to 3T3-L1 adipocytes. As shown Fig. 2D, palmytoyl LPE, palmytoyl LPG and palmytoyl LPI did not stimulate glucose uptake in 3T3-L1 adipocytes, suggesting that the head group of LPC may contribute to the structural selectivity in stimulation of glucose uptake by LPC in 3T3-L1 adipocytes.

FIG. 2A to 2D show the effects of LPC on the glucose uptake in 3T3-L1 adipocytes. FIG. 2A shows 3T3-L1 adipocytes grown in six-well plates were equilibrated in glucose-free Krebs-Ringer buffer for 1hr and incubated with LPC (0 to 30 µM) or insulin (10 nM) for 10 min. After these treatments, [¹⁴C] 2-deoxy-D-glucose uptake was measured for 10 min as described in Material Methods. FIG. 2B shows 3T3-L1 adipocytes were incubated with LPC (20 µM) for 0 to 20 min. FIG. 2C and 2D show relative [¹⁴C] 2-deoxy-D-glucose uptake in 3T3-L1 adipocytes incubated in the absence (control) or presence of equimolar concentrations (20 µM) of myristoyl lysophosphatidylcholine (14:0 LPC), palmytoyl lysophosphatidylcholine (16:0 LPC), stearoyl lysophosphatidylcholine (18:0 LPC), palmytoyl lysophsophatidylethanolamine (16:0 LPE), palmytoyl lyso-phosphatidylinositol (16:0 LPI), palmytoyl lysophosphatidylglycerol (16:0 LPG) for 10 min. Values are mean ± SE of three independent experiments performed in triplicate. **P* < 0.05 vs. basal.

### EXAMPLE 3

### LPC (not belonging to the present invention) stimulates GLUT4 translocation in 3T3-L1 adipocytes.

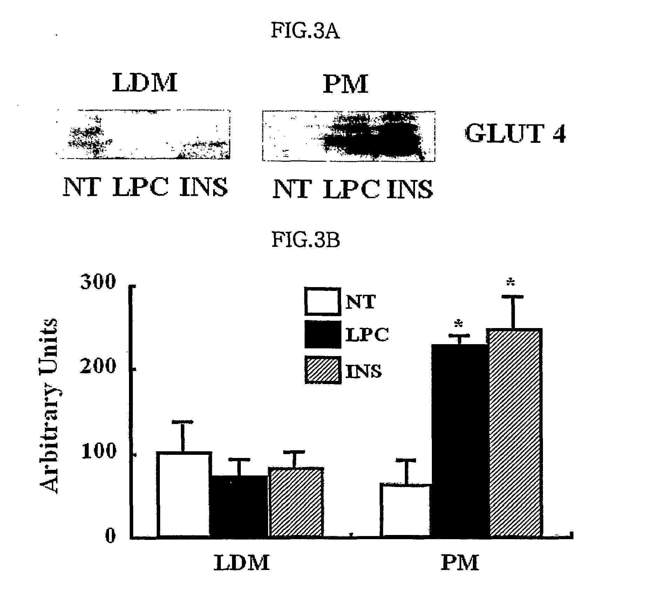

For assessing whether the ability of LPC to enhance glucose transport in 3T3-L1 adipocytes could be mediated by LPC-induced changes in the amounts of glucose transporter protein at the cell surface, the protein levels of GLUT4, the predominant glucose transporter isoforms expressed in 3T3-L1 adipocytes, was measured in PM fractions in the basal state or after treatment with LPC or insulin. LPC induced a significant increase in the PM content of GLUT4 proteins (180% of basal) like insulin (Fig. 3*A* and 3*B*). The results suggest that both insulin and LPC stimulate GLUT4 translocation and are consistent with the observation in glucose uptake experiments.

FIG 3A and 3B show LPC stimulates GLUT4 translocation in 3T3-L1 adipocytes. A) Effect of insulin on GLUT4 translocation to the plasma membrane (PM) in 3T3-L1 adipocytes. Low-density microsome, LDM. 3T3-L1 adipocytes were stimulated for 10 min with 100 nM insulin or 20 µM lysophospholipids. In each experiment, the relative increase or decrease in the integrated density value (IDV) of GLUT4 after stimulation with compounds is calculated. B) Quantitation of relative increases is depicted. Values are mean ± SE of three independent experiments performed in triplicate.**P* < 0.05.

### EXAMPLE 4

### LPC (not belonging to the present invention) stimulates glucose uptake via PKCδ activation.

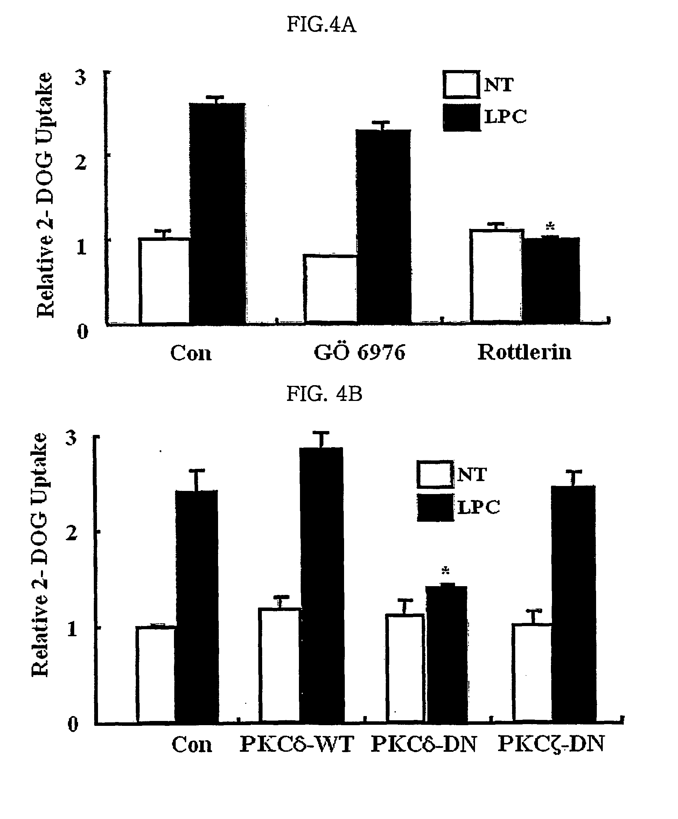

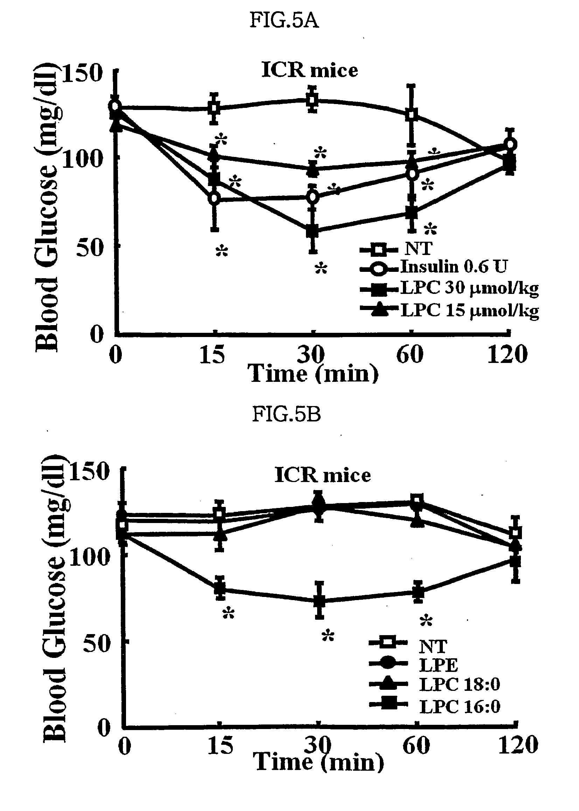

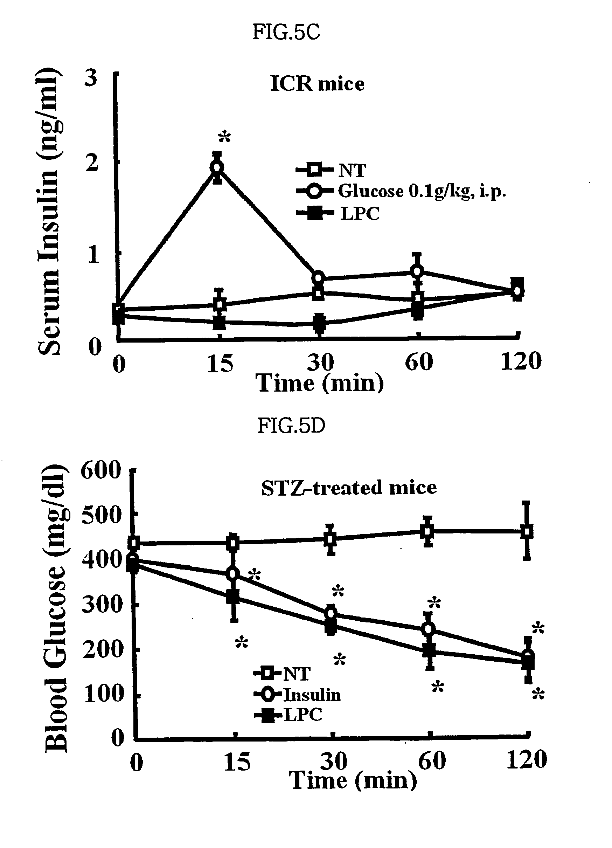

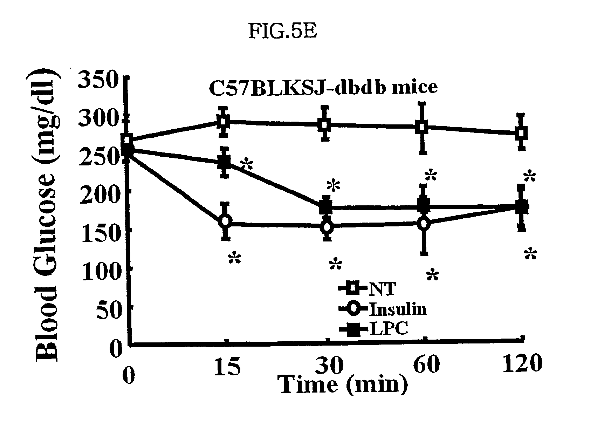

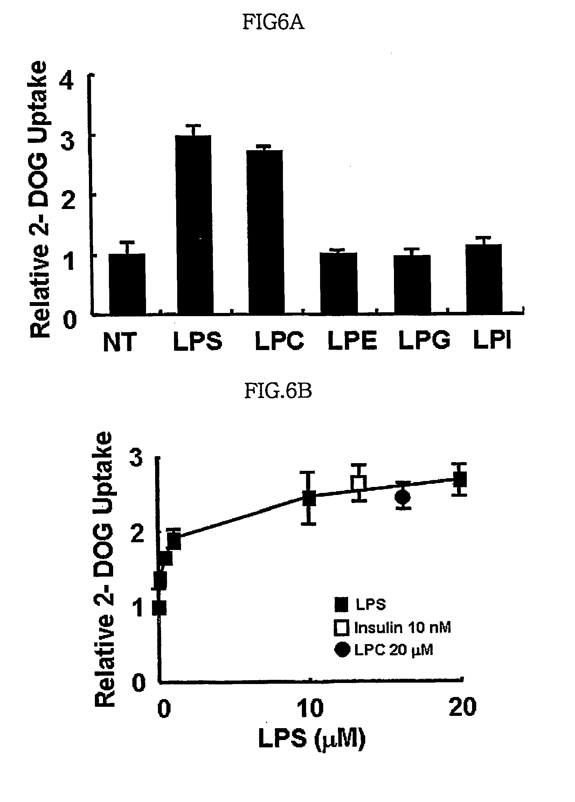

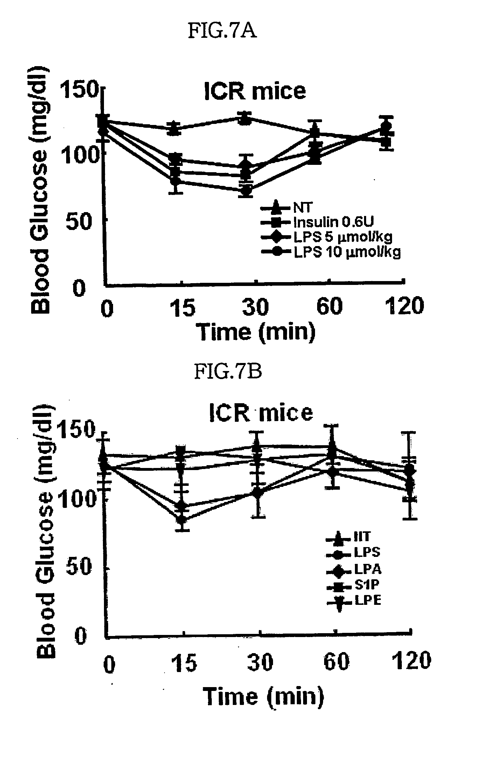

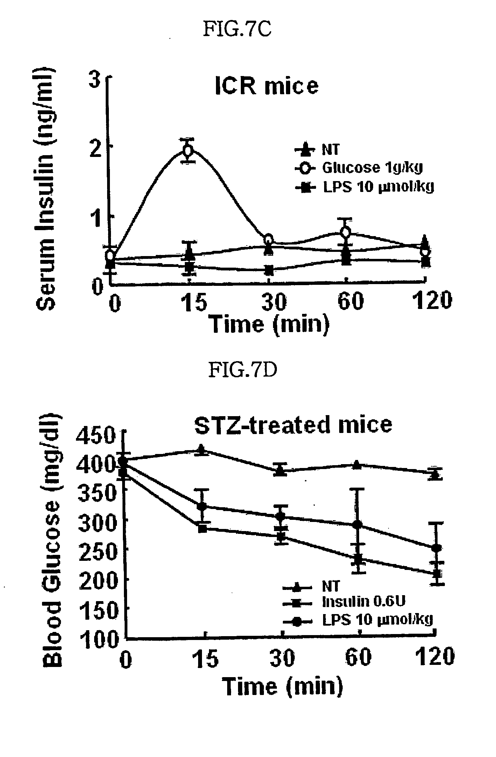

Insulin stimulation of glucose uptake in adiopocytes requires activation of IRS 1, PI 3-kinase and subsequent activation of AKT(Burgering, B. M., et al., Nature 376, pp.599-602, 1995; Baumann, C. A., et al., Nature 407, pp202-207, 2000). Thus, to determine whether increased glucose uptake in response to LPC was associated with insulin dependent signaling pathway, IRS1 and AKT phosphorylation was checked. As expected, 10 nM insulin treatments of 3T3-L1 adipocytes resulted in augmentation of IRS1 and AKT phosphorylation (supplement data). By contrast, LPC treatment of adipocytes had no effects on phosphorylation of IRS1 and AKT (supplement data). Because LPC has been shown to activate conventional and novel PKC in various cells(Chaudhuri, P., et al.,cell migration. Circ Res 97, 674-681, 2005), the involvement of these PKCs in the LPC-induced augmentation of glucose transport was assessed next. Pretreatment of 3T3-L1 adipocytes with 2 µM Gö6976, conventional PKC inhibitor, for 30 min did not alter LPC stimulation of glucose uptake. However, LPC-stimulated glucose uptake was completely inhibited by pretreatment with 10 µM rottlerin, an inhibitor of PKCδ (Fig. 4A)

To test the role of PKC more directly, we used an adenovirus expression system to overexpress specific PKC isoforms and dominant negative PKC isoforms in 3T3-L1 adipocytes. We assayed glucose uptake in 3T3-L1 adipocytes overexpressing the wild-type, dominant negative PKCδ or dominant negative PKCζ. The expression of the wild-type PKCδ induced slight increases in glucose transport activity of LPC-stimulated states, compared with that in control 3T3-L1 adipocytes. Expression of the dominant negative mutant of PKCδ reduced significant LPC-stimulated glucose transport activity. In contrast, overexpression of dominant negative PKCζ altered neither LPC-induced nor Insulin-induced glucose uptake (Fig. 4B). These findings demonstrate that a PKCδ could participate in LPC-induced glucose transport activation.

FIG. 4A and 4B show that LPC stimulates glucose uptake via PKCδ activation. FIG. 4A shows 3T3-L1 adipocytes grown in six-well plates were equilibrated in glucose-free Krebs-Ringer buffer for 1hr and were treated with 2 µM Gö6976, 10 µM rottlerin or buffer alone as indicated for 30 min. Then, cells were treated with vehicle (open bars) or 20 µM LPC (filled bars) for 10 min. After these treatments, [¹⁴C] 2-deoxy-D-glucose uptake was measured for 10 min as described in Material Methods. FIG. 4B shows expression levels PKCδ and PKCζ proteins. Lysates from control 3T3-L1 adipocytes and from those expressing PKCδ WT, PKCδ DN or PKCζ DN were immunoblotted with anti-PKCδ or PKCζ antibody (*Top*)*,* and glucose uptake measurement in 3T3-L1 adipocytes (*Bottom*)*.* Control 3T3-L1 adipocytes and 3T3-L1 adipocytes expressing PKCδ WT, PKCδ DN, or PKCζ DN were incubated with vehicle or 20 µM LPC or 10 nM insulin for 10 min. Values are mean ± SE of three independent experiments performed in triplicate.**P* < 0.05.

### EXAMPLE 7

### Effect of LPA on glucose uptake in 3T3-L1 adipocytes

### 7-1: Effects of LPA on the glucose uptake in 3T3-L1 adipocytes.

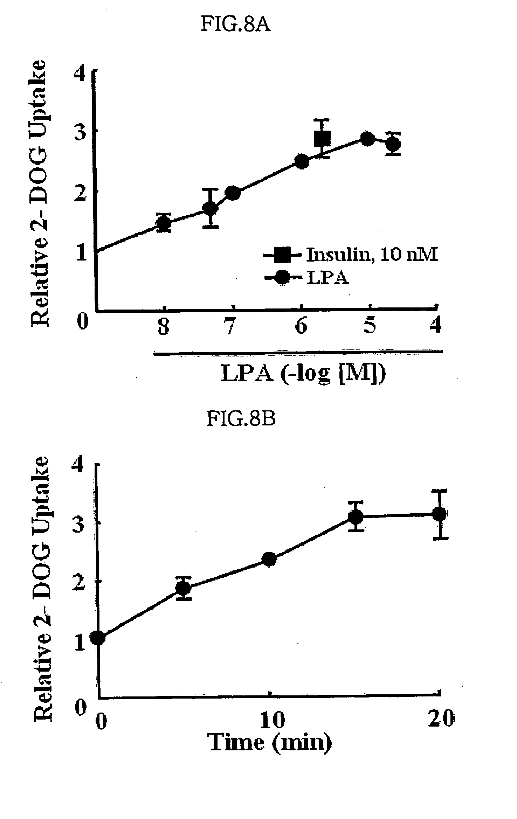

According to the substantially same method of EXAMPLE concerning LPC, the effect of LPA was tested in 3TS-L1 adipocytes, to show the result in FIG. 8A and 8B.

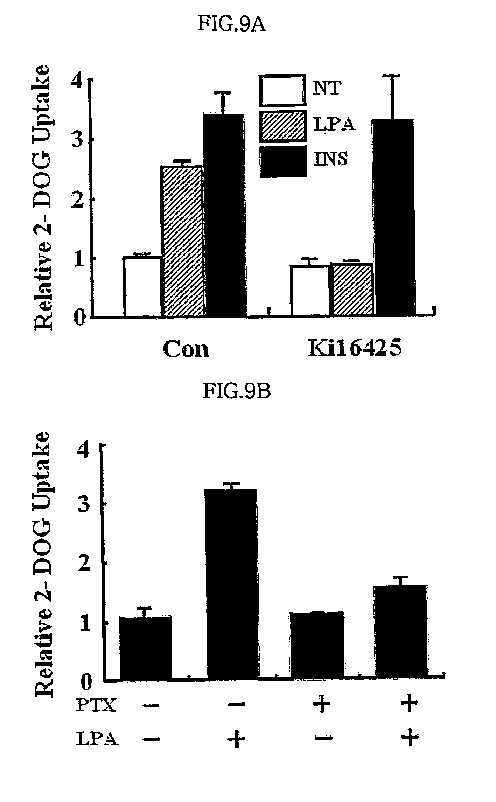

For investigating the effects of LPA on the glucose uptake, 3T3-L1 adipocytes were incubated in the presence of various concentrations of standard LPA for different times. LPA stimulated a time- and dose-dependent increase in glucose uptake in 3T3-L1 adipocytes. An initial statistically significant effect of LPA on glucose uptake was observed at the concentration of 1 µM and the maximal effect at 20 µM (Fig. 8A). With 20 µM LPA, glucose uptake was maximally increased after 10 min of incubation with LPA (Fig. 8B). FIG. 9*A* shows 3T3-L1 adipocytes grown in six-well plates were equilibrated in glucose-free Krebs-Ringer buffer for 1hr and incubated with LPA (0 to 20 µM) or insulin (10 nM) for 10 min. After these treatments, [¹⁴C] 2-deoxy-D-glucose uptake was measured for 10 min as described in Material Methods. FIG. 8*B* shows 3T3-L1 adipocytes were incubated with LPA (20 µM) for 0 to 20 min.

### 7-2: Signaling mechanisms in the stimulation of glucose uptake by LPA

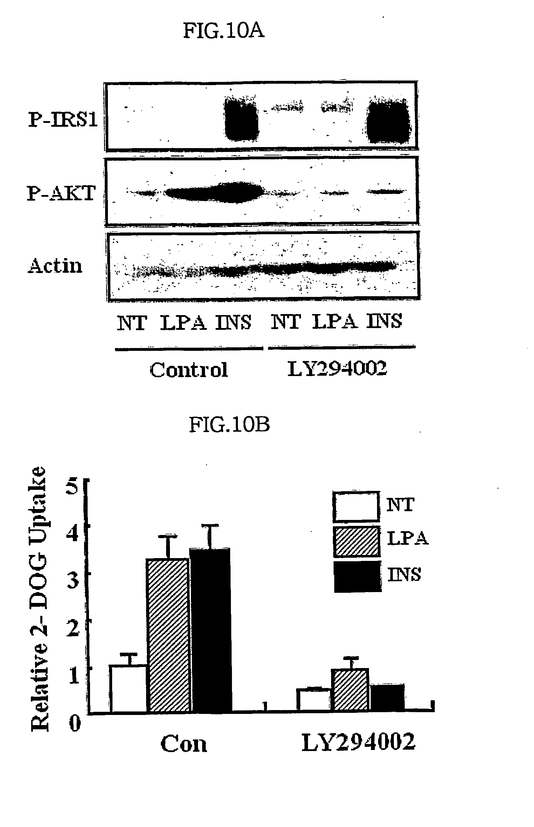

According to the substantially same method of EXAMPLE concerning LPC, the effect of LPA was tested in 3T3-L1 adipocytes, to show the result in FIG. 9A , and 9B, and FIG. 10A and 10B.

To investigated whether LPA affect glucose uptake via its receptor association we used LPA receptor antagonist, Ki16425. Fig. 9A shows that glucose uptake stimulation by LPA is completely inhibited by Ki 16425 pretreatment. Next, we check whether LPA activates LPA receptor which coupled to Gαi by using the Gαi inhibitor, pertussis toxin.

Fig. 9B shows that LPA stimulates glucose uptake via to Gαi activation.

It is well reported that insulin stimulated glucose uptake via PI3-kinase dependent signaling pathways. To investigate whether LPA enhances glucose uptake via PI3-kinase dependent signaling pathway, we checked Akt, the down stream signal of PI3-kinase, is affected by LPA treatment. Fig. 10*A* shows that LPA stimulated Akt phosphorylation. This phosphorylation is inhibited by PI3-kinase inhibitor, LY294002 pretreatment. Next, to test LPA actually stimulates glucose uptake via PI3-kinase signal pathway, we pretreated LY294002, and measured glucose uptake in 3T3-L1 adipocytes. Fig 10*B* shows that the stimulation of glucose uptake by LPA is dependent on PI3-kinase activation.

### 7-3: Glucose-lowering effect of LPA in mouse models.

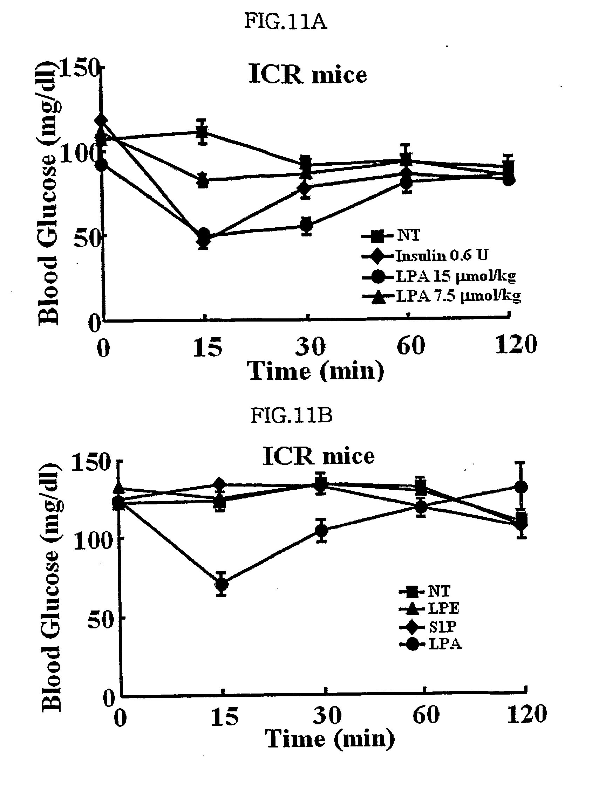

According to the substantially same method of EXAMPLE concerning LPC, the glucose-lowering effect of LPA was tested in mouse models, to show the result in FIG. 11A to 11D.

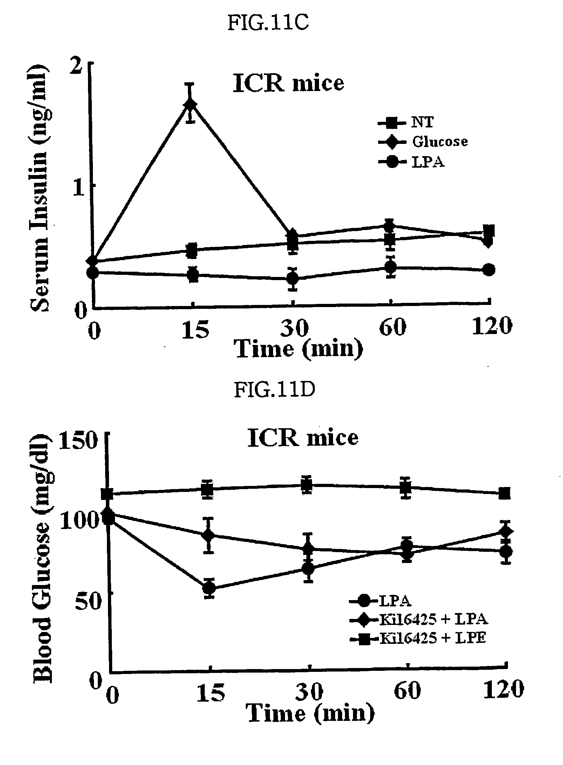

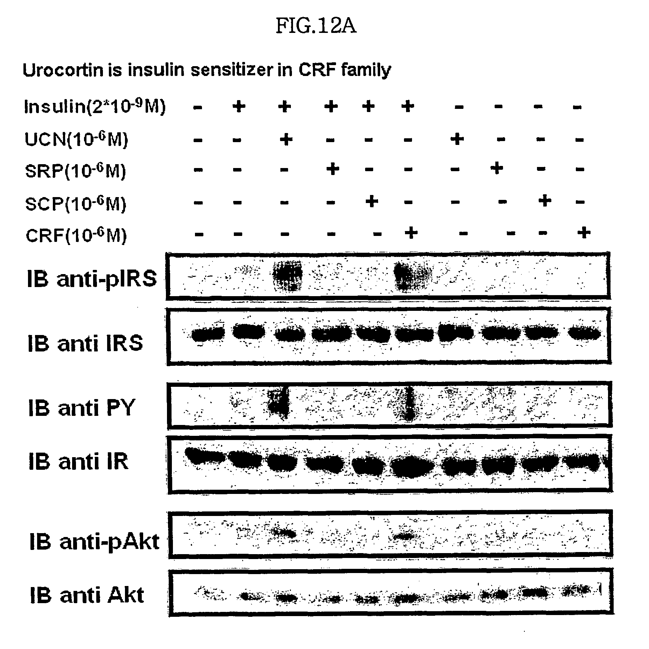

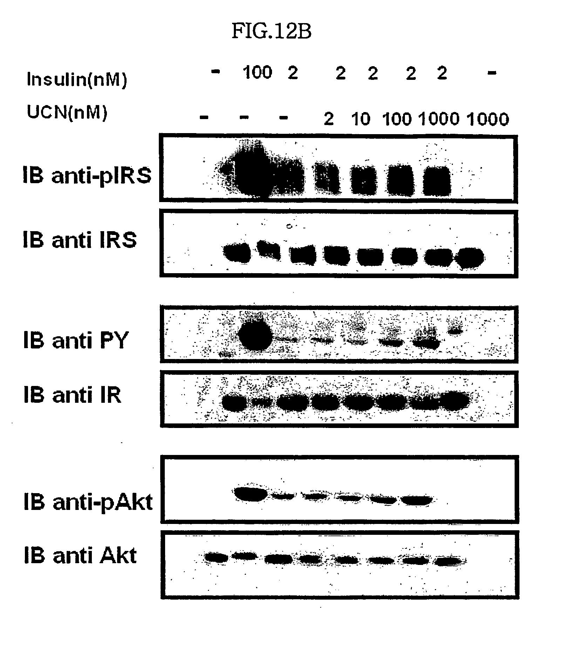

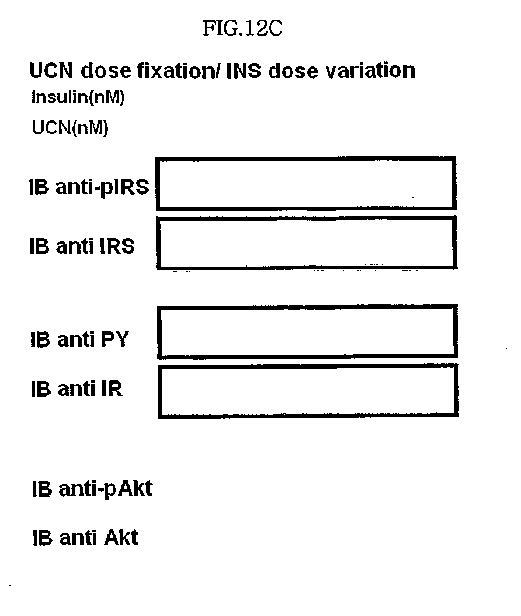

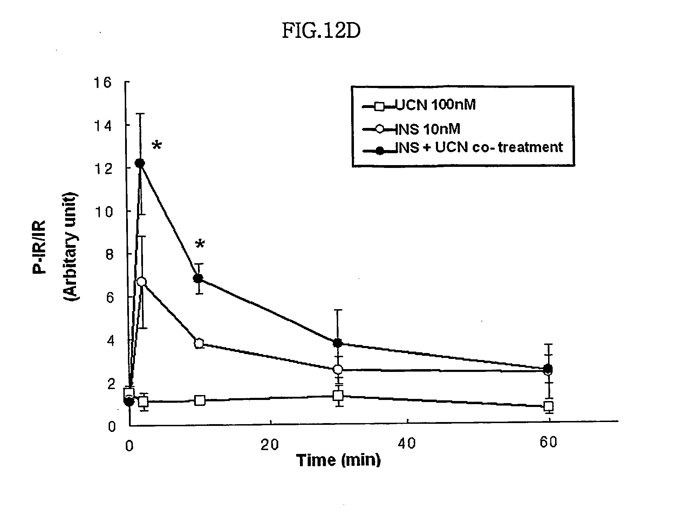

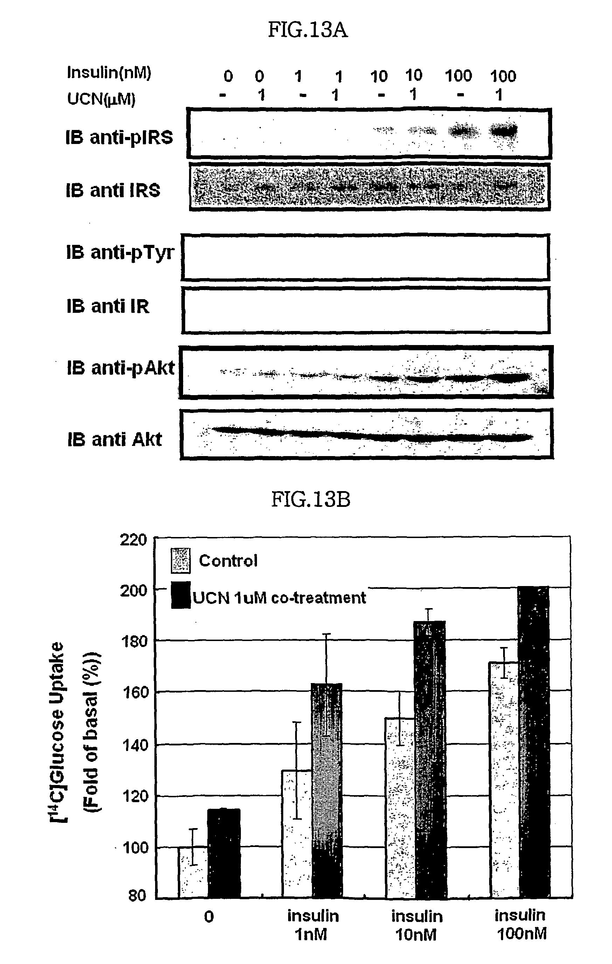

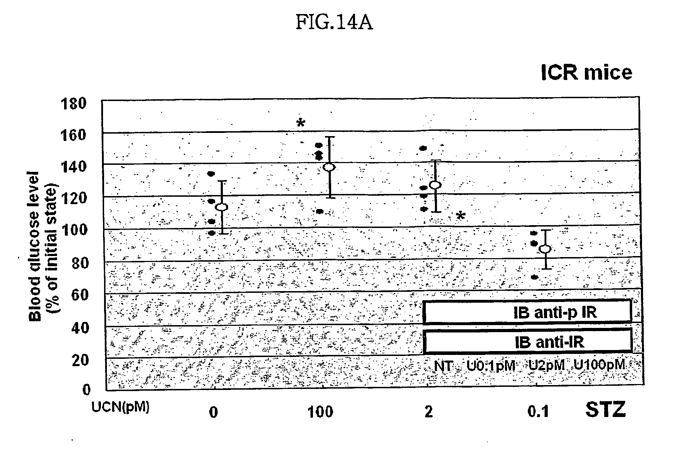

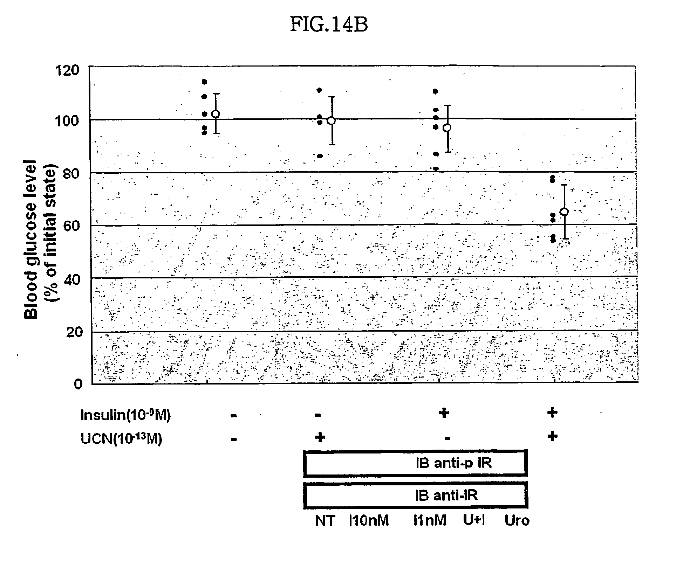

FIG 11A to 11B showed glucose lowering efficacy of intravenously administrated LPA in mouse models. Eight-week-old male mice were intravenously injected with PBS, insulin, LPA. Blood glucose was monitored after dosing (0 to 120 min). FIG. 11A showed serum insulin level in eight-week-old male mice after single intravenous injection of LPA. LPA lowered the level of blood glucose in normal mice dose-dependently. Other lysophospholipids such as SIP and LPE did not lower the blood glucose level in normal mice (FIG. 11 B). This effect was not due to changes in blood insulin levels (FIG. 11C).

Finally, we tested whether the glucose lowering effect by LPA is dependent on LPA receptor activation. Prior to administration of LPA, the LPA receptor inhibitor, Ki 16425 was injected. FIG 11D shows the glucose lowering effect by LPA is inhibited by LPA receptor inhibitor. From these data, LPA lowers the level of blood glucose dose-dependently and dose not affect insulin secretion. Furthermore, blood glucose lowering by LPA is mediated by LPA receptor activation. All animals had free access to water. Animal care was in accordance with institutional guidelines. All data are shown as means ± SE (n = 5-6).**P* < 0.05.

## Claims

1. A glucose uptake modulator comprising lysophosphatidic acid (LPA), for use in the treatment of diabetes or diabetic complications in a mammal in a need thereof, comprising administering to said mammal an effecting amount of said glucose uptake modulator.

2. The glucose uptake modulator for use according to claim 1, wherein the diabetes is insulin-dependent diabetes mellitus or noninsulin-dependent diabetes mellitus.

3. The glucose uptake modulator for use according to claim 2, wherein the diabetic complication is obesity, hyperlipidemia, arteriosclerosis, hypertension or heart disease.

4. The glucose uptake modulator for use according to claim 3, which further comprises administering to said mammal an effecting amount of a glucose uptake modulator in combination of at least a compound selected from the group consisting of insulin secretion enhancers, biguanides, and α-glucosidase inhibitors.

## Patentansprüche

1. Ein Glukoseaufnahmemodulator, welcher Lysophosphatidsäure (LPA) enthält, zur Verwendung bei der Behandlung von Diabetes oder diabetischen Komplikationen in einem Säuger, welcher Bedarf daran hat, umfassend das Applizieren einer wirksamen Menge des Glukoseaufnahmemodulators bei dem Säuger.

2. Der Glukoseaufnahmemodulator zur Verwendung gemäß Anspruch 1, wobei der Diabetes ein insulinabhängiger Diabetes mellitus oder ein nicht-insulinabhängiger Diabetes mellitus ist.

3. Der Glukoseaufnahmemodulator zur Verwendung gemäß Anspruch 2, wobei die diabetische Komplikation Fettsucht, Hyperlipidämie, Arteriosklerose, Bluthochdruck oder eine Herzerkrankung ist.

4. Der Glukoseaufnahmemodulator zur Verwendung gemäß Anspruch 3, welcher ferner das Applizieren an dem Säuger von einer wirksamen Menge eines Glukoseaufnahmemodulators in Kombination mit wenigstens einer Verbindung umfasst, welche ausgewählt wird aus der Gruppe bestehend aus Insulinsekretionsverstärkern, Biguaniden und α-Glukosidase-Inhibitoren.

## Revendications

1. Modulateur de la capture du glucose comprenant de l'acide lysophosphatidique (LPA), pour son utilisation dans le traitement du diabète ou des complications diabétiques chez un mammifère en ayant besoin, comprenant l'administration au dit mammifère d'une quantité efficace dudit modulateur de la capture du glucose.

2. Modulateur de la capture du glucose pour une utilisation selon la revendication 1, dans lequel le diabète est un diabète sucré insulinodépendant ou un diabète sucré non insulinodépendant.

3. Modulateur de la capture du glucose pour une utilisation selon la revendication 2, dans lequel la complication diabétique est l'obésité, l'hyperlipidémie, l'artériosclérose, l'hypertension ou la cardiopathie.

4. Modulateur de la capture du glucose pour une utilisation selon la revendication 3, qui comprend en outre l'administration au dit mammifère d'une quantité efficace d'un modulateur de la capture du glucose en combinaison avec au moins un composé choisi dans le groupe constitué des amplificateurs de la sécrétion d'insuline, des biguanides, et des inhibiteurs de l'α-glucosidase.
